# EUROPÄISCHE PATENTSCHRIFT

(11) **EP 1 124 593 B1**
(45) Veröffentlichungstag und Bekanntmachung des Hinweises auf die Patenterteilung: **05.06.2002**
(21) Anmeldenummer: 99953931.5
(22) Anmeldetag: 25.10.1999
(51) Int. Cl.: A61L 27/36

(54) **VERFAHREN ZUR PRÄPARATION VON KNOCHENMATERIAL**
METHOD FOR PREPARING BONE MATERIAL
PROCEDE POUR LA PREPARATION DE MATIERE OSSEUSE

(30) Priorität: 29.10.1998 DE 19849984
(43) Veröffentlichungstag der Anmeldung: 22.08.2001
(73) Patentinhaber: Tutogen Medical GmbH, 91077 Neunkirchen am Brand (DE)
(72) Erfinder: KÜBLER, Norbert, D-97076 Würzburg (DE)
(74) Vertreter: Manitz, Finsterwald & Partner
(86) Internationale Anmeldenummer: EP9908056
(87) Internationale Veröffentlichungsnummer: WO0025839

(56) Entgegenhaltungen:
- WO-A-96/39203
- US-A- 4 472 840
- US-A- 5 112 354
- GLOWACKI, J. ET AL.: "Demineralized Bone Implants" CLINICS PLASTIC SURGERY, Bd. 12, Nr. 2, April 1985 (1985-04), Seiten 233-241, XP000874409

## Beschreibung

Die Erfindung bezieht sich auf die Präparation und Gewinnung von demineralisiertem Knochenmaterial, das zur Wiederherstellung bei knöchernen Defekten in der Chirurgie geeignet ist.

Die Verwendbarkeit demineralisierten Knochenmaterials ist bereits bekannt. 1965 beschrieb M. R. Urist das osteoinduktive Potential von demineralisiertem Knochen nach intramuskulärer Implantation im Tierexperiment. Dieses Knochenmaterial enthält eine oder mehrere osteoinduktiv wirksame Substanzen wie z.B. die sogenannten bone morphogenetic proteins (BMPs), die eine Knochenregeneration in einem knöchernen Defekt stimulieren können (Lit. Urist, M.R.: Bone Formation by Autoinduction. Science 150: 893,1965.).

Eine Verbesserung dieses osteoinduktiven Knochenmaterials wurde über die Jahre hinweg schrittweise erreicht. Durch die Erkenntnis, daß eine im phosphatgepufferten Medium geführte Aktivierung von endogenen Knochenenzymen, die einen Abbau von osteoinduktiven Proteinen bewirken, durch verschiedene Enzyminhibitoren wie Natriumazid, Jodessigsäure, Jodacetamid, N-Ethylmaleinimid, Phenylmethyl-sulfonylfluorid und p-Chlorquecksilberbenzoat, unterdrückt werden kann, ohne gleichzeitig die Autolyse von Knochenzellen zu beeinträchtigen, führte schließlich zu einem speziellen Herstellungsverfahren, mit dem ein sogenannter AAA-Knochen, ein autolysierter, Antigen extrahierter, allogener Knochen, präpariert werden konnte (vgl. Kübler N., et al., J.Oral Maxillofac. Surg., 51: 1346-1357, 1993.). Dieser AAA-Knochen hat bei gleichzeitig reduzierter Allo-Antigenität osteoinduktive Eigenschaften. Die Reduzierung der antigenen Eigenschaften wird durch Behandlung der zellulären Bestandteile durch Autolyse sowie deren Extraktion mit Chloroform-Methanol erreicht. Hierbei wird Knochenmaterial, das einem Verfahren zur Herstellung als AAA-Knochen unterzogen wird, aus Verstorbenen gewonnen.

Die Gewinnung des Ausgangsmaterials für das demineralisierte Knochenmaterial aus Verstorbenen ist durch den Einfluß von nach dem Tode einsetzenden Zersetzungsvorgängen beschränkt. Diese Autolyse setzt unmittelbar post mortem ein und zerstört die für die beabsichtigte Wirkung des Ersatzmaterials erforderlichen Wirksubstanzen im entnommenen Knochen. Eine Gewinnung von geeignetem Knochen ist damit bislang nur unmittelbar bzw. wenige Stunden post mortem möglich, wie z.B. aus Multiorganspendern. Die Zahl an Multiorganspendern ist sehr gering und erlaubt keine Gewinnung von Ausgangsmaterial für Material im Sinne der Erfindung mit dem Zwecke einer zuverlässigen und gesicherten Versorgung von Chirurgen. Andere Verstorbene kommen darüber hinaus als Spender praktisch nicht in Frage, da das Einholen einer Erlaubnis zur Gewebeentnahme innerhalb des kurzen Zeitfensters für eine Gewebeentnahme nicht möglich ist. Es konnte jedoch gezeigt werden, daß die Konzentration und die Wirksamkeit der das Knochenwachstum stimulierenden Substanzen unter bestimmten Umständen bis zu 24 Stunden post mortem erhalten bleibt und dadurch eine Entnahme sowohl aus hirntoten Spendern wie aus normal Verstorbenen möglich ist.

Es ist die Aufgabe der vorliegenden Erfindung, ein verbessertes Verfahren zur Präparation von Knochenmaterial zu schaffen, das den Heilungsprozeß nach einer Implantation beschleunigt.

Die Lösung dieser Aufgabe erfolgt durch die Merkmale des Anspruchs 1 und insbesondere dadurch, daß die Verweilzeit des Knochenmaterials in der Pufferlösung 24 Stunden nicht überschreitet. Überraschenderweise hat sich nämlich herausgestellt, daß die bislang als vorteilhaft angesehene Verweilzeit von 72 Stunden weder erforderlich noch vorteilhaft ist, um das Knochenmaterial zu präparieren und um die für den Heilungsprozeß wesentlichen BMPs zu erhalten. Mit dem erfindungsgemäßen Verfahren wird die Verträglichkeit des Knochenmaterials im lebenden Gewebe des Empfängers des Knochenimplantates verbessert und die Wirksamkeit bzw. Freisetzung der im knöchernen Träger enthaltenen Substanzen, die eine Knochenregeneration in einem Knochendefekt stimulieren können, werden verbessert, wodurch eine beschleunigte Heilung erreicht wird.

Erfindungsgemäß bleibt die natürlichen Knochensubstanz als Träger der Wirkstoffe und als Gerüstsubstanz zum biomechanisch korrekten Einbau weitgehend erhalten. Die vorliegende Erfindung verbessert die Behandlung des Leichenknochens jedoch derart, daß bestimmte Behandlungsschritte verkürzt und damit der Zeitverlauf des Abbaus der biologisch aktiven Inhaltsstoffe im Knochen vermindert werden.

Erfindungsgemäß besitzen die das Knochenwachstum stimulierenden, natürlichen Substanzen, die aus der Gerüstsubstanz des erfindungsgemäß behandelten Knochens erhalten werden, eine erhöhte Wirksamkeit gegenüber dem bisherigen Verfahren. Der Nachweis der verbesserten Wirksamkeit erfolgt durch eine Implantationstest mit Ratten. Die Implantation des erfindungsgemäß behandelten Knochens in die Muskulatur von Ratten führt zur Erzeugung von Knochen- und Knorpelvorläuferzellen sowie zur Bildung von ausdifferenzierten Knochen- und Knorpelzellen. Die Bildung dieser Zellen ist semi-quantitativ auswertbar und ist durch die Menge bzw. durch den zeitlichen Anstieg der alkalischen Phosphataseaktivität ein Maß für die Aktivität der biologischen Inhaltsstoffe.

Weiter bleibt erfindungsgemäß die natürliche Knochensubstanz erhalten, die vom Empfängerorganismus als verträglich erkannt wird und im Verlaufe der Einheilung in körpereigenes Gewebe umgebaut wird. Ein wesentliches Element der Erfindung besteht dabei darin, daß die Chemikalien zur Bearbeitung des Ausgangsmaterials biologisch nicht stören und die Einheilung nicht behindern.

Gemäß der Erfindung bewirken die eingesetzten Chemikalien zur Demineralisierung der Gerüstsubstanz und zur Extraktion der zellulären Bestandteile gleichzeitig eine chemische Sterilisation, so daß eine akzidentelle Kontamination des Knochens durch Mikroben, entstanden im Verlaufe der Knochenentnahme oder während des Herstellungsverfahrens, durch das Behandlungsverfahren selbst beseitigt wird. Die Gewinnung des Knochens aus dem Leichenspender innerhalb einer 24-stündigen Frist erfolgt unter aseptischen Bedingungen. Damit kann eine Verkeimung durch Sporen im Spendergewebe ausgeschlossen werden. Eine Verkeimung durch vegetative Keime, wie sie in einem nachfolgenden Bearbeitungsverfahren zufällig oder durch nicht steril / aseptisch geführte Arbeitsgänge erfolgen kann, wird durch die verfahrensgemäße Anwendung der eingesetzten Chemikalien beseitigt. Eine terminale Sterilisation wie z.B. durch Hitze oder Gas, wie sie zur Herstellung anderer pharmazeutischer Produkte eingesetzt wird, ist nicht obligatorisch, so daß die Inhaltsstoffe des demineralisierten Knochens vollständig erhalten werden können.

Vorteilhafte Ausführungsformen der Erfindung sind in der Beschreibung und in den Unteransprüchen beschrieben.

So beträgt bei einer ersten vorteilhaften Variante des erfindungsgemäßen Verfahrens die Verweilzeit nicht mehr als 10 Stunden, vorzugsweise etwa 6 Stunden. Durch eine solche, im Vergleich zum Stand der Technik drastisch reduzierte Verweilzeit in der Pufferlösung wird die biologische Aktivität des erhaltenen Knochenmaterials deutlich gesteigert, ohne daß es jedoch erforderlich oder vorteilhaft wäre, eine erhöhte Konzentration an Enzyminhibitoren zuzugeben.

Auch ist es vorteilhaft, wenn der Demineralisierung eine Entfettung vorausgeht. Hierbei ist jedoch eine Mischung aus Chloroform und Methanol als verwendetes Lösungsmittel physiologisch bedenklich, da bereits geringe Rückstände das Einheilungsverhalten beeinträchtigen können und darüber hinaus die Verwendungsmöglichkeit von Chloroform in einem pharmazeutischem Herstellungsverfahren aus arbeitsschutzrechtlichen Gründen mit erheblichen Beschränkungen verbunden ist. Der Ersatz des vorbekannten Entfettungsmittelgemisches Chloroform / Methanol durch andere Entfettungsmittel, z.B. Methanol allein, Chloroform allein, Ethanol, Äther, Azeton und andere Niedrigsieder und Gemische daraus bevorzugt Äther, ermöglicht eine verbesserte, rückstandsfreie Entfernung aus dem Knochengewebe und eine verbesserte Verträglichkeit des Knochenersatzmaterials.

Besonders vorteilhaft ist es ferner, wenn das erhaltene Knochenmaterial am Ende der Prozeßfolge einer Gamma-Sterilisation unterworfen wird, da hierdurch ohne Einfluß auf die biologische Aktivität eine Sterilisierung des Materials erfolgen kann.

Nachfolgend wird die vorliegende Erfindung beispielhaft anhand einer vorteilhaften Ausführungsvariante beschrieben.

Bei dem erfindungsgemäßen Verfahren zur Präparation von Knochenmaterial sowie der Herstellung von knöchernen Defekten in der Chirurgie wird zunächst humaner, kortikaler Knochen, z.B. vom Femur, Tibia, Humerus oder kortikaler Knochen des Beckenkamms sowie des Craniums von geeigneten Spendern unter sterilen Bedingungen innerhalb von 6 Stunden post mortem und bei -80°C gelagert. Der gefrorene Knochen wird zur Bearbeitung in sterilem Wasser mit 2 mmol/l Natriumazid aufgetaut. Das Natriumazid dient als Enzyminhibitor um einen Abbau der osteoinduktiven Knochenmatrixproteine zu unterbinden. Die Knochenenden bzw. Gelenkansätze werden entfernt und der Knochen wird befreit von anhaftendem, nicht ossärem Gewebe und eventuell vorhandenes Knochenmark wird entfernt. Für Zwischenlagerungen während dieses Vorgangs wird der Knochen in destilliertem Wasser mit 2 mmol/l Natriumazid, 2 mmol/l N-Ethylmaleinimid und 0,1 mmol/l Benzamidin-Hydrochlorid bei 4°C gelagert und auf diese Weise eine enzymatische Aktivität unterbunden. Alternativ kann die Lagerung beispielsweise auch in 10 mmol/l Natriumazid und 3mmol/l N- Ethylmaleinimid bei 4°C erfolgen.

Anschließend erfolgt eine Entfettung in einem Chloroform/Methanol-Gemisch (1:1) bei Raumtemperatur über etwa 4 Stunden.

Nach einer Verdampfungszeit (Evaporierungszeitraum) von etwa 1 Stunde wird der Knochen zur Demineralisation in 0,6 mol/l Salzsäure bei 4°C eingelegt. Der Grad der Demineralisation ist abhängig von der Zeitdauer und dem Verhältnis zwischen dem mineralischen Gewicht und dem Volumen der Salzsäure. Dauer der Salzsäurebehandlung und damit Grad der Demineralisation liegen zwischen wenigen Stunden für eine Oberflächendemineralisierung und bis zu 30 Stunden für eine vollständige Demineralisation. Dabei löst die Salzsäure auch säurelösliche Proteine heraus wie Knochen-Sialoprotein, Osteopontin, Osteonectin, Osteocalcin, und Thrombospondin. Die Behandlung mit Salzsäure ermöglicht die Diffusion der BMPs in das Empfängergewebe post implantanonem und erleichtert Osteoinduktion und Resorption durch Makrophagen und Osteoklasten.

Nach der Demineralisation wird der Knochen erneut einer Säuberung auf eventuelle Gewebereste auf der Knochenoberseite unterzogen und in sterilem, destilliertem Wasser bei 4°C für 30 - 60 Minuten gewaschen. Durch Inkubation in 0,1 mol/l Phosphat-Puffer, pH 7,4, mit 3 mmol/l N-Ethylmaleinimid und 10 mmol/l Natriumazid zur Erhaltung der osteoinduktiven Matrixproteine wird ein autolytischer Abbau der Knochenzellen durchgeführt. Die Behandlung erfolgt bei 37°C unter Schütteln über etwa 6 Stunden in einem Wasserbad. Ein Wechsel der Pufferlösung kann erfolgen. Anschließend wird der Knochen in sterilem, deionisiertem Wasser für 2 bis 4 Stunden bei 4°C gerührt. Das Wasser wird für diesen Vorgang zweimal gewechselt.

Es folgt eine Schrumpfung der Kollagenfibrillen und die Extraktion hochmolekularer Proteoglykane mittels 6 mol/l Lithiumchlorid sowie die Extraktion von Protein-Polysacchariden mit geringem Molekulargewicht wie Biglykanen, Dekorin, Fibromodulin etc. durch 0,3 mol/l Calciumchlorid. Die Lösung enthält 3 mmol/l Natriumazid und die Extraktion wird über 24 Stunden bei 4°C durchgeführt.

Der Knochen wird anschließend in sterilem destillierten Wasser für 12 Stunden bei 4°C gewaschen, wobei ein mehrfacher Wasserwechsel durchgeführt wird. Lipide sowie Lipoproteine der Zellmembran werden über 24 Stunden mittels einer 1:1 Mischung aus Chloroform-Methanol extrahiert. Ein zusätzlicher Effekt dieser Behandlung besteht in der Inhibition sowie Extraktion endogener, BMPs abbauender Enzyme. Nach Abgießen der Chloroform-Methanollösung wird der Knochen unter sterilen Bedingungen getrocknet. Schließlich wird der Knochen wiederum mit sterilem, deionisierten Wasser für 4 Stunden bei 4°C gewaschen und anschließend tiefgefroren und danach für 10 Tage lyophilisiert und schließlich steril verpackt. Hieran kann sich noch eine Gamma-Sterilisation anschließen.

In Stichworten gestaltet sich das erfindungsgemäße Herstellungsverfahren für Knochenstücke wie folgt:
1 Bei -80°C tiefgefroren gelagerten Knochen in Aqua dest. mit 2,0 mM Natriumazid auftauen.
2 Knochenenden entfernen.
3 Knochen von Weichgeweben befreien.
4 Knochenstücke mittels Kürette von Knochenmark befreien.
5 Knochen in gewünschte Größe sägen.
6 Knochen mit starkem, kaltem Wasserstrahl von Knochenmark befreien, Knochen nicht austrocknen lassen, in Aqua dest, 4°C, mit 2,0 mM Natriumazid 2,0 mM N-Ethylmaleinimid und 0,1 mM Benzamidin-HCl lagern.
7 Entfetten durch ein Bad in Chloroform/Methanol 1:1 bei RT, 4 h.
8 Evaporieren für ca 1 h.
9 Demineralisierung mit 0,6 M HCl bei 4°C für 2 bis 16 h je nach gewünschtem Demineralisierungsgrad (am nächsten Tag Röntgenkontrolle).
10 Oberste Schicht des demineralisierten Knochens und noch anhaftendes Weichgewebe entfernen.
11 Waschen mit Aqua dest. bei 4°C für 1 h.
12 Knochen für 6 h in 0,1 M Phosphatpuffer, pH 7,4 mit 3,0 mM N-ethylmaleinimid und 10,0 mM Natriumazid bei 37°C im Wasserbad inkubieren.
13 Mit Aqua dest, für 2-4 h bei 4°C waschen und das Aqua dest. zweimal wechseln.
14 Für 24 Std mit 6.0 M LiCl/0,3 M CaCl₂ mit 3,0 mM Natriumazid bei 4°C inkubieren.
15 Mit Aqua dest. 12 bis 24 h bei 4°C waschen und das Aqua dest. mindestens zweimal wechseln.
16 Abtöten von Keimen und Sporen durch Desinfektion bzw. Chemo-Sterilisation sowie Extraktion zellulärer Abbauprodukte mittels Chloroform/Methanol (1:1) bei Raumtemperatur für 24 h.
17 Evaporieren für ca. 2-3 h.
18 Mit sterilem Aqua dest. für 4 h bei 4 °C waschen, das Wasser zweimal wechseln.
19 Lyophilisieren für 10 Tage; anschliessend Sterilproben prüfen.
20 Knochenstücke steril verpacken.
21 Eventuell Sterilisierung mittels Gamma-Sterilisation bei 3 MRad.

Es sei darauf hingewiesen, daß die oben genannten Schritte 10 bis 18 grundsätzlich in ihrer Reihenfolge vertauscht werden können.

Ein Herstellungsverfahren für Knochenpulver gestaltet sich gemäß der Erfindung wie folgt:
1 Knochen in Aqua dest. Mit 2,0 mM Natriumazid (0,13 g auf 1l) auftauen.
2 Knochen von Weichgewebe befreien.
3 Knochen in kleine Stücke sägen.
4 Knochenstücke von Knochenmark befreien und unter kaltem Wasser nochmals säubern.
5 Knochen nicht austrocknen lassen, in Aqua dest, 4°C, mit 10,0 mM Natriumazid 3,0 mM N-Ethylmaleinimid lagern.
6 Mit der Knochenmühle unter Verwendung von flüssigem Stickstoff grob mahlen (ca. 2,0 mm Korngröße).
7 Entfetten durch ein Bad in Chloroform/Methanol 1:1 bei RT, für 1 bis 4 h.
8 Evaporieren für ca 1 h.
9 Auf die gewünschte Größe (ca 0,5 mm Korngröße) mahlen unter Kühlung, z.B. durch flüssigen Stickstoff.
10 Demineralisierung mit 0.6 N HCl bei 4°C über Nacht (am nächsten Tag Röntgenkontrolle).
11 Waschen mit Aqua dest. bei 4° C für 1 h.
12 Pulver für 6 Stunden in 0,1 M Phosphatpuffer, pH 7,4 mit 3,0 mM N-ethylmaleinimid und 10,0 mM Natriumazid bei 37° C im Wasserbad inkubieren.
13 Mit Aqua dest. 10 mM Natriumazid und 3,0 mM N-Ethylmaleinimid für 4 h bei 4°C waschen und das Aqua dest. zweimal wechseln.
14 Für 24 h mit 6,0 M LiCl /0,3 M CaCl₂ mit 3,0 mM Natriumazid bei 4°C inkubieren.
15 Mit Aqua dest. 10 mM Natriumazid und 3,0 mM N-Ethylmaleinimid den ganzen Tag bei 4°C waschen und Aqua dest. mindestens zweimal wechseln.
16 Abtöten von Keimen und Sporen durch Desinfektion bzw. Chemo-Sterilisation sowie Extraktion zellulärer Abbauprodukte mittels Chloroform/Methanol (1:1) bei Raumtemperatur für 24 h.
17 Evaporieren für ca 1 h.
18 Mit sterilem Aqua dest. für 1 h bei 4° C waschen.
19 Lyophilisieren.
20 Nach 10 Tagen Sterilproben prüfen.
21 Pulver steril verpacken.
22 Eventuell Sterilisierung mittels Gamma-Sterilisation bei 3 MRad.

Auch hier sind die oben genannten Schritte 11 bis 18 grundsätzlich in ihrer Reihenfolge vertauschbar.

Die nachfolgenden Tabellen zeigen den Einfluß verschiedener Verfahrensparameter auf die Osteoblastenaktivität (alkalische Phosphatase (AP)). In den Tabellen steht U für Einheiten und n für Anzahl von Proben. Die Angabe 3/4 in der Spalte Histologie/Osteoinduktion bedeutet 3 Tiere von 4 zeigen Osteoinduktion. Entsprechendes gilt für die weiteren Angaben in den Spalten Histologie/Osteoinduktion und Chondroinduktion in vitro.

Tabelle 1A zeigt die Abhängigkeit der Osteoblastenaktivität (alkalische Phosphataseaktivität (AP)) nach 10- bzw. 15- tägiger Implantation sowie der osteoinduktiven Potenz nach 4-wöchiger heterotroper, d.h. intramuskulärer Implantation in Ratten von der Expositionsdauer des AAA-Knochens gegenüber Phosphatpuffer in Kombination mit den genannten Enzyminhibitoren (3,0 mmol/l N-Ethylmaleinimid und 10 mmol/l Natriumazid bei 37°C) während der Autolyse:

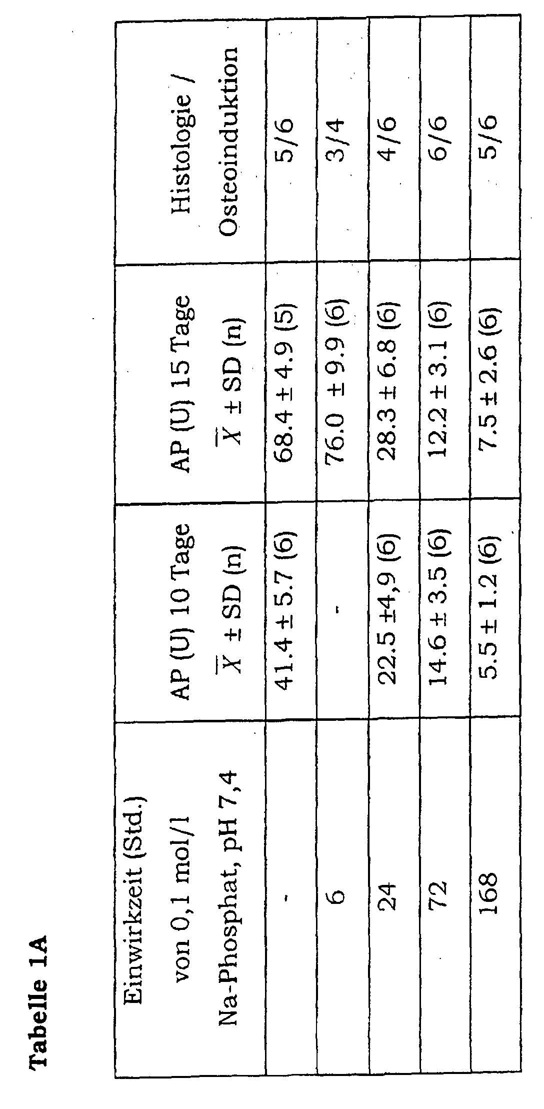

Wie Tabelle 1A zeigt, wirkt sich eine über 6 Stunden hinausgehende Einwirkzeit in der Phosphatpufferlösung nicht vorteilhaft auf die Osteoblastenaktivität aus. Zudem kann durch eine Verringerung der bislang gewählten Einwirkzeit von 72 Stunden auf 6 Stunden eine Steigerung der Osteoblastenaktivität auf das über 6-fache erzielt werden (nach 15-tägiger Implantation).

Tabelle 1B zeigt die Abhängigkeit der Osteoblastenaktivität (alkalische Phosphatase (AP)) nach 10- bzw. 15-tägiger Implantation sowie der osteoinduktiven Potenz nach 4- wöchiger heterotroper, d.h. intramuskulärer Implantation in Ratten von der Expositionsdauer des AAA-Knochens gegenüber der neutralen Phosphatpufferlösung in Kombination mit unterschiedlichen Konzentrationen an Enzyminhibitoren:

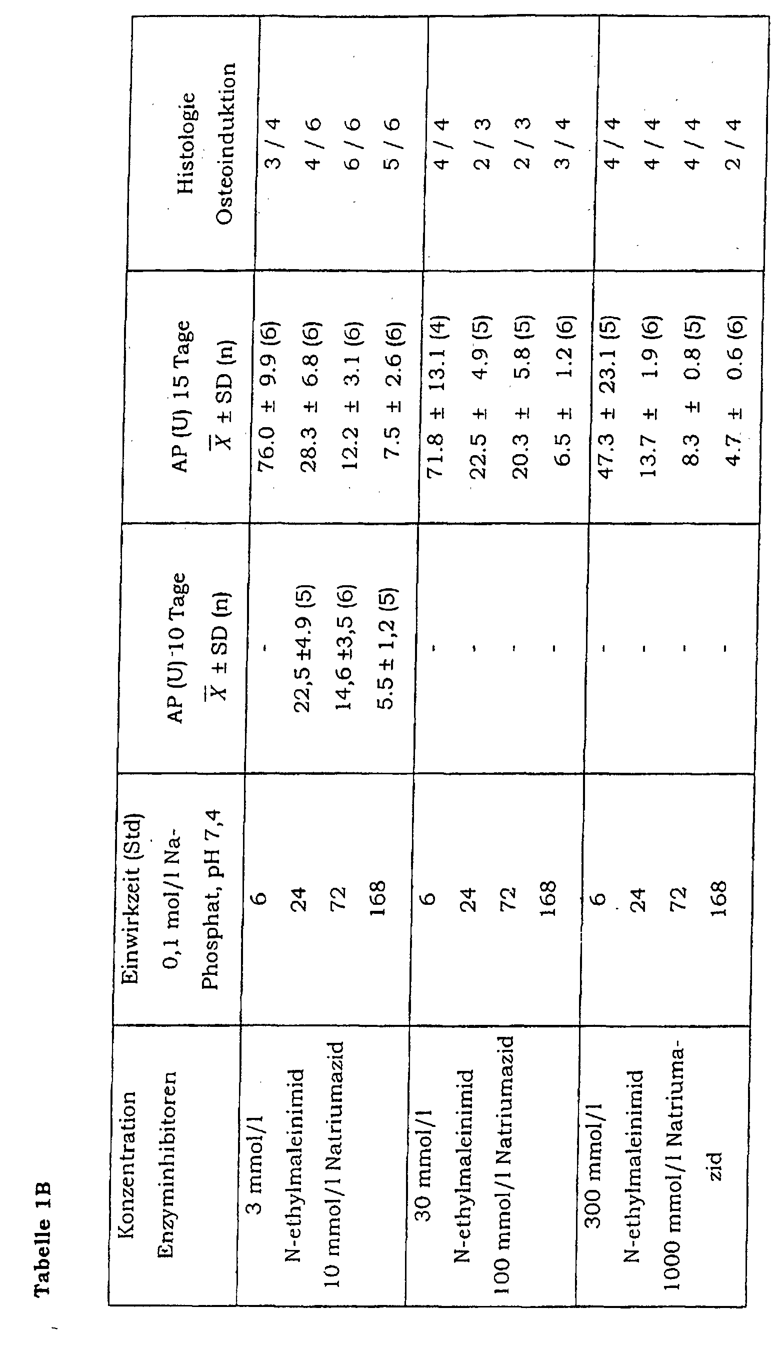

Wie Tabelle 1B zeigt, wird die Osteoblastenaktivität durch eine erhöhte Konzentration an Enzyminhibitoren während der Autolyse in neutraler Phosphatpufferlösung nicht gesteigert. Auch wirkt sich eine über 6 Stunden hinausgehende Einwirkzeit in der Phosphatpufferlösung nicht positiv auf die Osteoblastenaktivität (nach 15-tägiger Implantation) aus.

Tabelle 2 zeigt den Einfluß von Lösungsmittelgemischen, eingesetzt zur Entfettung und Chemosterilisation, auf die Osteoblastenaktivität (alkalische Phosphatase (AP) und Osteoinduktion) nach 4-wöchiger heterotroper, d.h. intramuskulärer Implantation in Ratten sowie der Chondroinduktion nach 14-tägiger Einwirkung auf neonatale Rattenmuskulatur in vitro:

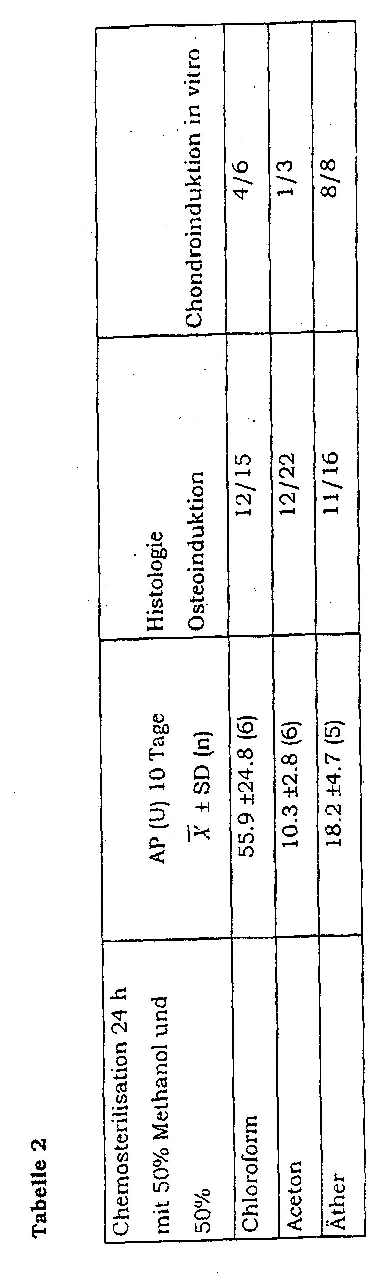

Schließlich zeigt Tabelle 3 die Abhängigkeit der Osteoblastenaktivität (alkalische Phosphatase (AP)) nach 10- bzw. 15-tägiger Implantation sowie der osteoinduktiven Potenz nach 4-wöchiger heterotroper, d.h. intramuskulärer Implantation in Ratten von verschiedenen Sterilisationsmethoden. Wie zu erkennen ist, wird durch eine Gamma-Sterilisation mit bis zu 3 MRad die Osteoblastenaktivität nicht beeinflußt. Somit ist eine Gamma-Sterilisation mit dieser Dosis den übrigen dargestellten Sterilisationsarten deutlich vorzuziehen (Eine Konservierung in Cialit® wird nach heutigem Wissensstand als obsolet angesehen, obgleich auch Cialit® in einer Konzentration von 1:2000 keinen nachteiligen Effekt auf die Osteoblatenaktivität und osteoinduktive Aktivität besitzt):

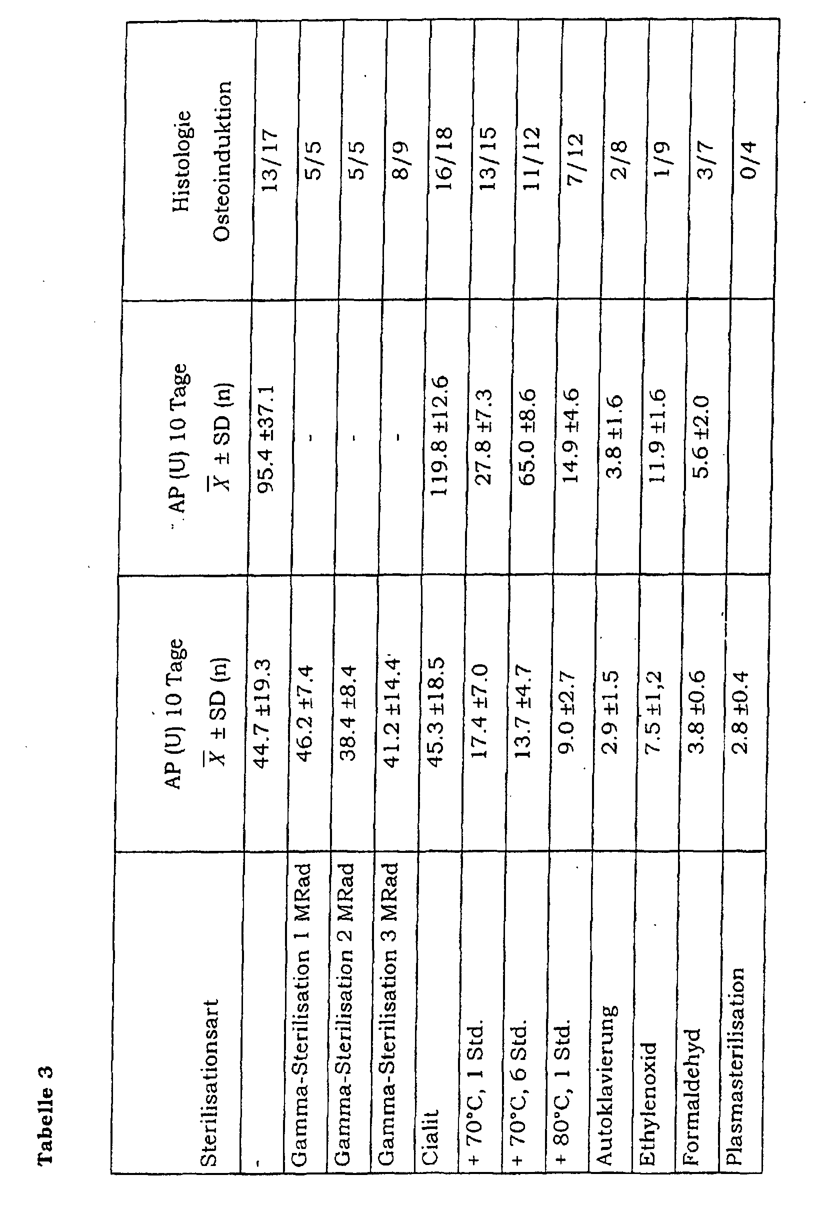

## Patentansprüche

1. Verfahren zur Präparation von Knochenmaterial zur Wiederherstellung von knöchernen Defekten in der Chirurgie, umfassend folgende Schritte:
- Demineralisieren von vorzugsweise kortikalem Knochenmaterial,
- autolytischer Abbau der Knochenzellen des demineralisierten Knochenmaterials unter Erhaltung der osteoinduktiven Matrixproteine durch Waschen in einer Phosphatpufferlösung unter Zuführung von Enzyminhibitoren,
**dadurch gekennzeichnet, daß**
die Verweilzeit in der Pufferlösung etwa 6 bis 24 Stunden beträgt.

2. Verfahren nach Anspruch 1,
**dadurch gekennzeichnet, daß**
die Verweilzeit nicht mehr als etwa 10 Stunden beträgt.

3. Verfahren nach Anspruch 1,
**dadurch gekennzeichnet, daß**
der Demineralisierung eine Entfettung, vorzugsweise unter Verwendung einer Methanolmischung, vorausgeht.

4. Verfahren nach Anspruch 1,
**dadurch gekennzeichnet, daß**
sich an die Demineralisierung eine Chemosterilisation, vorzugsweise unter Verwendung einer Methanolmischung, anschließt.

5. Verfahren nach Anspruch 3 oder 4,
**dadurch gekennzeichnet, daß**
die Methanolmischung neben Methanol Äther oder Chloroform aufweist.

6. Verfahren nach Anspruch 1,
**dadurch gekennzeichnet, daß**
das erhaltene Knochenmaterial einer Gamma-Sterilisation unterworfen wird.

7. Verfahren nach Anspruch 1,
**dadurch gekennzeichnet, daß**
das Knochenmaterial vor der Demineralisierung unter Verwendung von flüssigem Stickstoff zu Pulver gemahlen wird.

8. Verfahren nach Anspruch 1,
**dadurch gekennzeichnet, daß**
das Knochenmaterial vor der Demineralisierung ohne Erhitzung über 40°C zu Pulver gemahlen wird.

9. Knochenmaterial, erhältlich durch ein Verfahren nach zumindest einem der vorstehenden Ansprüche.

## Claims

1. Method for the preparation of bone material for the surgical restoration of osseous defects comprising the following steps:
- demineralisation of preferably cortical bone material,
- autolytic decomposition of the bone cells of the demineralised bone material while retaining the osteo-inductive matrix proteins by washing in a phosphate buffer solution with the supply of enzyme inhibitors,
**characterised in that**
the dwell time in the buffer solution amounts to about 6 to 24 hours.

2. Method in accordance with claim 1,
**characterised in that**
the dwell time does not amount to more than about 10 hours.

3. Method in accordance with claim 1,
**characterised in that**
the demineralisation is preceded by degreasing, preferably using a methanol mixture.

4. Method in accordance with claim 1,
**characterised in that**
a chemical sterilisation follows the demineralisation, preferably using a methanol mixture.

5. Method in accordance with claim 3 or 4,
**characterised in that**
the methanol mixture has ether or chloroform in addition to methanol.

6. Method in accordance with claim 1,
**characterised in that**
the bone material which is obtained is subjected to gamma sterilisation.

7. Method in accordance with claim 1,
**characterised in that**
the bone material is ground to a powder with the use of liquid nitrogen prior to the demineralisation.

8. Method in accordance with claim 1,
**characterised in that**
the bone material is ground to a powder without heating to above 40°C prior to the demineralisation.

9. Bone material obtainable by a method in accordance with at least one of the preceding claims.

## Revendications

1. Procédé pour la préparation de matière osseuse pour la reconstitution de défauts osseux en chirurgie, comprenant les étapes suivantes :
- déminéralisation de matière osseuse, de préférence corticale,
- décomposition autolytique des cellules osseuses de la matière osseuse déminéralisée en conservant les protéines matricielles ostéoinductives par lavage dans une solution tampon de phosphate avec un apport d'inhibiteurs enzymatiques,
**caractérisé en ce que** le temps de séjour dans la solution tampon s'élève approximativement à 6 à 24 heures.

2. Procédé selon la revendication 1, **caractérisé en ce que** le temps de séjour ne dépasse pas environ 10 heures.

3. Procédé selon la revendication 1, **caractérisé en ce que** la déminéralisation est précédée par un dégraissage, de préférence en utilisant un mélange de méthanol.

4. Procédé selon la revendication 1, **caractérisé en ce que** la déminéralisation est suivie d'une chémo-stérilisation, de préférence en utilisant un mélange de méthanol.

5. Procédé selon l'une ou l'autre des revendications 3 et 4, **caractérisé en ce que** le mélange de méthanol comprend de l'éther ou du chloroforme en plus du méthanol.

6. Procédé selon la revendication 1, **caractérisé en ce que** la matière osseuse obtenue est soumise à une stérilisation gamma.

7. Procédé selon la revendication 1, **caractérisé en ce que** la matière osseuse est moulue avant la déminéralisation en utilisant de l'azote liquide pour former une poudre.

8. Procédé selon la revendication 1, **caractérisé en ce que** la matière osseuse est moulue avant la déminéralisation sans chauffage au-dessus de 40°C pour former une poudre.

9. Matière osseuse, susceptible d'être obtenue par un procédé selon l'une au moins des revendications précédentes.
